# EUROPÄISCHE PATENTANMELDUNG

(11) **EP 1 245 195 A1**
(43) Veröffentlichungstag der Anmeldung: **02.10.2002**
(21) Anmeldenummer: 01810319.2
(22) Anmeldetag: 29.03.2001
(51) Int. Cl.: A61B 17/30, A61F 2/16

(54) **Chirurgisches Instrument**

(71) Anmelder: GRIESHABER & CO. AG SCHAFFHAUSEN, CH-8203 Schaffhausen (CH)
(72) Erfinder: Attinger, Jürg, 8260 Stein am Rhein (CH); Hedinger, Heinrich, 8217 Wilchingen (CH)
(74) Vertreter: Althoff, Gerhard

(57) **Zusammenfassung**

Es wird ein chirurgisches Instrument, insbesondere ophthalmologisches Klemminstrument (5) vorgeschlagen, welches zwei etwa parallel zueinander orientierte längliche Klemmarme (10,20) umfasst, die an dem hinteren Basisteil (2) miteinander wirkverbunden und an dem vorderen Funktionsteil (1) mit relativ zueinander bewegbaren Klemmteilen (15,25) versehen sind.

An den beiden Klemmarmen (10,20) ist in axialer Richtung im Abstand zu dem vorderen Funktionsteil (1) eine mit zwei gegenüberliegend zueinander angeordneten Druckstücken (16,26) versehene Betätigungseinheit (30) angeordnet, mittels welcher beim Zusammendrücken der beiden Druckstücke eine inverse Bewegungsfunktion und infolge davon ein Spreizen der beiden vorderen Klemmteile (15,25) entgegen der federelastischen Rückstellkraft der Klemmarme (10,20) erreichbar ist.

## Beschreibung

Die Erfindung bezieht sich auf ein chirurgisches Instrument, insbesondere ophthalmologisches Klemminstrument mit zwei etwa parallel zueinander orientierten länglichen Klemmarmen, welche an dem hinteren Basisteil miteinander wirkverbunden und an dem vorderen Funktionsteil mit relativ zueinander bewegbaren Klemmteilen versehen sind.

Bei den sich fortlaufend weiterentwickelnden mikrochirurgischen Techniken, insbesondere aber bei ophthalmologischen Eingriffen werden an die jeweils zu verwendenden Instrumente zum Schneiden oder dergleichen sowie zum Spreizen und zum Klemmen (Festhalten) unter Beibehaltung einer präzisen Funktion sowie einer einfachen Handhabung verhältnismässig hohe Anforderungen gestellt.

Bei chirurgischen Eingriffen am Auge eines Lebewesens sind diese Anforderungen von entscheidender Bedeutung, insbesondere aber dann, wenn in Abhängigkeit des jeweiligen Eingriffs der Ophthalmologe auf engstem Raum mit mehreren Instrumenten arbeitet, beziehungsweise arbeiten muss. Hierbei ist es erschwerend und auch von besonderem Nachteil, wenn der Ophthalmologe beispielsweise eine zusammengelegte und zu implantierende künstliche Linse durch eine Inzision einführen oder aber den zur Freilegung der Operationsstelle eingeschnittenen Gewebelappen mittels zusammengedrückter Pinzette in Bezug auf den Operationsbereich manuell in aufgeklappter Stellung halten muss und infolge dessen in den Bewegungen zusätzlich eingeschränkt ist.

Der Erfindung liegt die Aufgabe zugrunde ein chirurgisches Instrument der eingangs genannten Art zu schaffen, mittels welchem unter Beibehaltung einer sicheren und sterilen Handhabung der Gegenstand, beispielsweise eine zu implantierende künstliche Linse oder ein eingeschnittener Gewebelappen erfasst, geklemmt und ohne aktive manuelle Betätigung beliebig fixierbar ist.

Das erfindungsgemässe chirurgische Instrument ist gekennzeichnet durch zwei im Abstand zu dem vorderen Funktionsteil gegenüberliegend zueinander an den Klemmarmen angeordnete Druckstücke, mittels welcher beim Zusammendrücken derselben eine inverse Bewegungsfunktion und infolge davon ein Spreizen der beiden vorderen Klemmteile entgegen der federelastischen Rückstellkraft der beiden Klemmarme erreichbar ist.

Weitere Merkmale der Erfindung ergeben sich aus der nachstehenden Beschreibung in Verbindung mit der Zeichnung und den einzelnen Patentansprüchen.

Ein Ausführungsbeispiel der Erfindung wird nachstehend anhand der Zeichnung beschrieben. Es zeigt:
- **Fig.1**: ein in räumlicher Ansicht sowie in grösserem Massstab dargestelltes chirurgisches Instrument mit zwei länglichen Klemmarmen und einer damit wirkverbundenen Betätigungseinheit;
- **Fig.2**: das in Draufsicht dargestellte chirurgische Instrument gemäss Fig.1 mit den beiden Klemmarmen und der damit wirkverbundenen Betätigungseinheit;
- **Fig.3**: das in Ansicht dargestellte chirurgische Instrument mit der Betätigungseinheit sowie zwei im vorderen Bereich an den beiden Klemmarmen angeordneten Klemmteilen in teilweise geöffneter Stellung;
- **Fig.4**: das in Ansicht dargestellte chirurgische Instrument gemäss Fig.3 mit den im vorderen Bereich in der geöffneten Endstellung dargestellten Klemmteilen der beiden Klemmarme; und
- **Fig.5**: die als Längsschnitt dargestellte und mit den beiden Klemmarmen in Wirkverbindung stehende Betätigungseinheit.

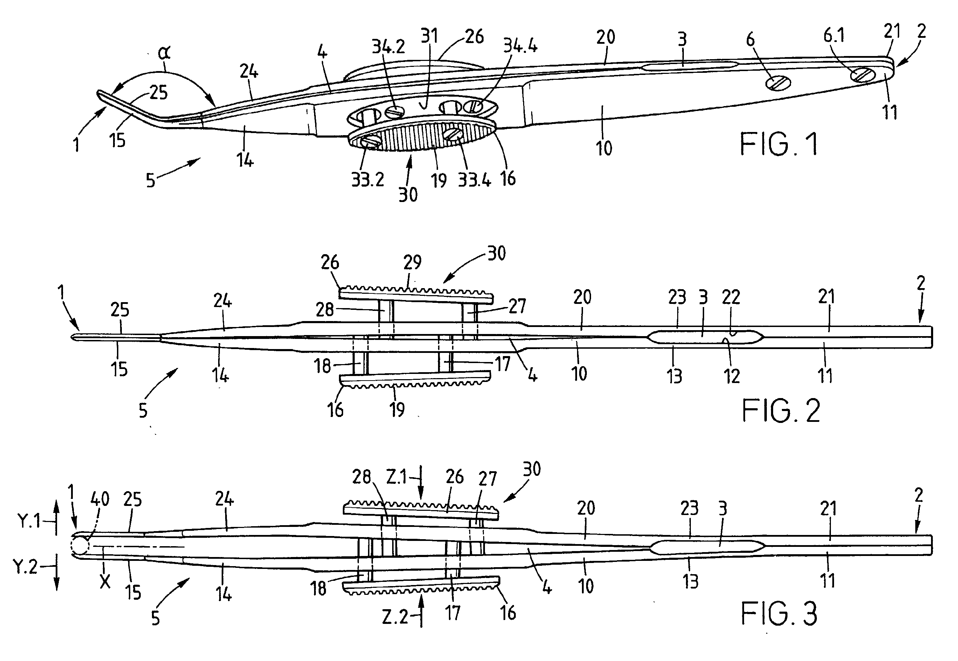

Fig.1 zeigt ein räumlich sowie in grösserem Massstab dargestelltes chirurgisches Instrument 5, welches insbesondere zur Durchführung ophthalmologischer Eingriffe ausgebildet ist. Das etwa in Form einer Pinzette ausgebildete chirurgische Instrument 5 umfasst zwei längliche Klemmarme 10 und 20 sowie eine damit in Wirkverbindung stehende Betätigungseinheit 30. Die mit zwei plättchenförmigen Druckstücken 16 und 26 versehene Betätigungseinheit 30 ist derart ausgebildet, dass diese beim Zusammendrücken eine inverse Bewegungsfunktion der am vorderen Ende der Klemmarme 10 und 20 angeordneten Klemmteile 15 und 25 bewirkt.

Die beiden Klemmarme 10 und 20 sind am vorderen Ende 1 (Funktionsspitze) mit dem Klemmteil 15 und 25 und am gegenüberliegenden hinteren Ende 2 (Klemmarmbasis) je mit einem Endstück 11 und 21 versehen. Die beiden Endstücke 11 und 21 sind im Bereich der einander zugewandten Innenflächen (nicht bezeichnet) bis zu einer Ausnehmung 3 flach aneinander liegend miteinander verbunden. Im dargestellten Ausführungsbeispiel sind die beiden Endstücke 11 und 21 mittels zwei im Abstand zueinander angeordneter Schrauben 6 und 6.1 miteinander befestigt. Die Befestigung der beiden Endstücke 11 und 21 kann auch mit anderen Mitteln, beispielsweise durch eine geeignete Nietoder Schweissverbindung erfolgen.

Die beiden am vorderen Ende der Klemmarme 10,20 angeordneten Klemmteile 15 und 25 sind bei dem in Fig.1 dargestellten Ausführungsbeispiel relativ zu den nicht bezeichneten Oberkanten der beiden vorderen Teilstücke 14 und 24 nach oben abgebogen ausgebildet.

Bei dem in Fig.1 dargestellten Ausführungsbeispiel sind die beiden Klemmteile 15 und 25 jeweils an dem vorderen Teilstück 14 und 24 unter stumpfem Winkel α an dem jeweiligen Klemmarm 10 oder 20 angeformt. Der Winkel α kann etwa in der Grössenordnung zwischen 110° bis 175° gewählt werden, wobei der Winkel α bei einer bevorzugten Ausführungsform 130° beträgt. Bei einer nicht dargestellten Ausführungsform besteht jedoch auch die Möglichkeit, dass die beiden Klemmteile 15 und 25 in Bezug auf die vorderen Teilstücke 14 und 24 nach unten abgebogen sind. Eine weitere Ausführungsform der beiden vorderen Klemmteile 15 und 25 besteht darin, dass diese in Abhängigkeit der Verwendung in nicht näher dargestellter Weise als geradlinige Verlängerung der Klemmarme 10 oder 20 ausgebildet sind.

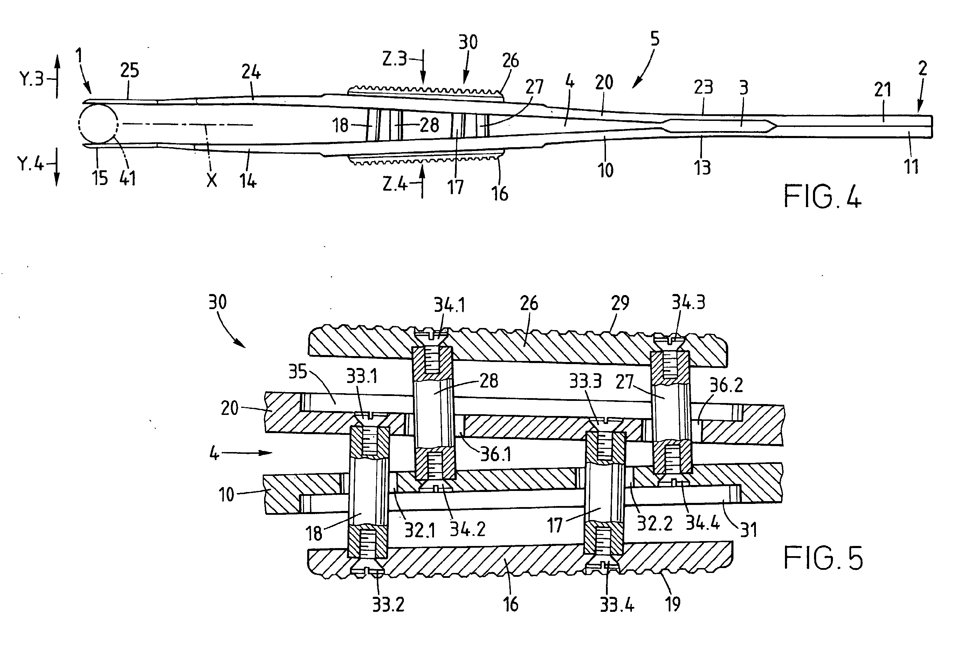

In Fig.1 sind die beiden Klemmteile 15 und 25 der Klemmarme 10 und 20 in geschlossener Stellung dargestellt, welche mittels der Betätigungseinheit 30 durch eine relativ zueinander orientierte Bewegung zum Erfassen und Halten eines Gegenstandes gespreizt oder geöffnet werden können. Die für eine sichere Handhabung durch den Chirurgen mit entsprechend ausgebildeten Druckstücken 16 und 26 versehene und mit den beiden Klemmarmen 10 und 20 wirkverbundene Betätigungseinheit 30 wird später in Verbindung mit Fig.5 beschrieben.

Die mit den beiden Klemmarmen 10 und 20 wirkverbundene Betätigungseinheit 30 ist in Längsrichtung gesehen, vorzugsweise etwa mittig zwischen der Ausnehmung 3 mit den beiden federelastisch wirkenden Wandteilen 13 und 23 und den beiden vorderen Klemmteilen 15 und 25 angeordnet.

Bei dem in Fig.1 bis Fig.5 dargestellten Ausführungsbeispiel sind die beiden mit der Funktionsspitze 1 und der Klemmarmbasis 2 versehenen Klemmarme 10 und 20 im Profilquerschnitt als rechteckiger Profilstab ausgebildet. Es besteht jedoch auch die Möglichkeit, dass die Klemmarme 10 und 20 beispielsweise als Rundstab oder dergleichen ausgebildet sind.

In Fig.2 ist das chirurgische Instrument 5 in Draufsicht dargestellt und man erkennt die beiden in Längsrichtung etwa parallel zueinander angeordneten Klemmarme 10 und 20 sowie die damit in Wirkverbindung stehende Betätigungseinheit 30. Die beiden Klemmarme 10 und 20 sind am Übergang zu den beiden miteinander verbundenen Endstücken 11 und 21 je mit einer Aussparung 12 und 22 versehen, welche in zusammengebautem Zustand die Ausnehmung 3 bilden. Die beiden Klemmarme 10,20 sind derart ausgebildet und zueinander angeordnet, dass die beiden vorderen Teilstücke 14,24 mit den Klemmteilen 15,25 bespielsweise infolge der Formgebung gegeneinander gedrückt werden. Mittels der Betätigungseinheit 30 können die beiden Klemmarme 10 und 20 entgegen der federelastischen Rückstellkraft der im hinteren Bereich der beiden Aussparungen 12 und 22 vorgesehenen Wandteile 13 und 23 im vorderen Bereich der Teilstücke 14,24 und Klemmteile 15,25 zum Erfassen eines nicht dargestellten Gegenstandes relativ zueinander beziehungsweise zu einer in Fig.3 und Fig.4 eingezeichneten theoretischen Symmetrieachse X bewegt beziehungsweise gespreizt werden.

In Fig.3 und Fig.4 ist das chirurgische Instrument 5 mit den beiden Klemmarmen 10,20 und die damit wirkverbundene und die Teile 16,17,18 sowie 26,27,28 umfassende Betätigungseinheit 30 in Ansicht dargestellt. Bei der in Fig.3 dargestellten und beliebig gewählten Zwischenstellung ist der eine Klemmarm 10 mittels der Teile 26,27,28 in Pfeilrichtung Z.1 und der andere Klemmarm 20 mittels der Teile 16,17,18 in Pfeilrichtung Z.2 bewegt. Bei den gemäss Pfeilrichtung Z.1 und Z.2 orientierten Bewegungen wird jeweils das eine vordere Klemmteil 15 gemäss Pfeilrichtung Y.2 und das andere vordere Klemmteil 25 gemäss Pfeilrichtung Y.1 relativ zu der theoretischen Symmetrieachse X zum Erfassen und Halten eines schematisch dargestellten Gegenstandes 40 geöffnet beziehungsweise gespreizt.

Bei der in Fig.4 dargestellten Stellung sind die beiden Klemmarme 10 und 20 gemäss Pfeilrichtung Z.3 und Z.4 bis in eine Endstellung bewegt, in welcher die beiden Druckstücke 16 und 26 an den zugewandten Oberflächen der Klemmarme 10,20 anliegen. Bei den gemäss Pfeilrichtung Z.3 und Z.4 orientierten Bewegungen wird jeweils das eine vordere Klemmteil 15 gemäss Pfeilrichtung Y.4 und das andere vordere Klemmteil 25 gemäss Pfeilrichtung Y.3 in Bezug auf die theoretische Symmetrieachse X zum Erfassen und Halten eines schematisch dargestellten Gegenstandes 41 geöffnet beziehungsweise gespreizt.

Der in Fig.3 und Fig.4 zwischen den beiden Klemmteilen 15 und 25 angeordnete Gegenstand 40 oder 41 kann beispielsweise eine künstliche Linse sein, welche zum Implantieren in nicht dargestellter Weise entlang einer theoretischen Symmetrieachse zusammengelegt zwischen den beiden Klemmteilen 15 und 25 gehalten ist.

Nach Freigabe des Gegenstandes 40 oder 41 beziehungsweise der Linse werden die beiden Klemmteile 15 und 25 sowie die einzelnen mit den Klemmarmen 10 und 20 wirkverbundenen Teile der Betätigungseinheit 30 in die Ausgangsstellung gemäss Fig.2 zurückbewegt. Die selbsttätige Schliessbewegung der beiden Klemmarme 10,20 wird im wesentlichen durch die federelastisch wirkende Rückstellkraft der im Bereich der Ausnehmung 3 etwa als Blattfeder ausgebildeten Wandteile 13 und 23 erreicht.

Die beiden infolge der Aussparung 12 und 22 hinsichtlich der Wanddicke reduzierten Wandteile 13 und 23 sind so ausgebildet und dimensioniert, dass lediglich eine geringfügige Haltekraft auf den erfassten Gegenstand 40 oder 41, beispielsweise in Form der Linse oder des Gewebelappens oder dergleichen wirkt. Eine unbeabsichtigte und zerstörende Verformung und/oder Quetschung des erfassten Gegenstandes infolge der federelastischen Rückstellkraft der Klemmarme 10 und 20 wird somit verhindert. Mit dem erfindungsgemässen Instrument 5 können beliebige Bewegungen, insbesondere um die theoretische Längsachse orientierte Rotationsbewegungen durchgeführt werden.

In Fig.5 ist die Betätigungseinheit 30 in grösserem Massstab sowie als Längsschnitt dargestellt und man erkennt die beiden durch den Längsschlitz 4 gespreizt dargestellten Klemmarme 10 und 20. Das erste Druckstück 26 ist mittels im Abstand zueinander angeordneter Zwischenglieder 27 und 28 mit dem ersten Klemmarm 10 wirkverbunden. Das zweite Druckstück 16 ist mittels im Abstand zueinander angeordneter Zwischenglieder 17 und 18 mit dem zweiten Klemmarm 20 wirkverbunden. Bei dem in Fig.5 dargestellten Ausführungsbeispiel sind die zylindrischen Zwischenglieder 17 und 18 mittels Schrauben 33.1 bis 33.4 an dem zweiten Druckstück 16 sowie an dem zweiten Klemmarm 20 und die zylindrischen Zwischenglieder 27 und 28 mittels Schrauben 34.1 bis 34.4 an dem ersten Druckstück 26 sowie an dem ersten Klemmarm 10 befestigt.

An dieser Stelle wird darauf hingewiesen, dass die beispielsweise in den Figuren 1 und 5 als Schraubverbindung dargestellte Befestigung der einzelnen Elemente auch durch andere Verbindungsglieder, beispielsweise in Form von Nieten oder aber durch eine geeignete Schweissverbindung erfolgen kann.

Die mit dem zweiten Druckstück 16 und dem zweiten Klemmarm 20 wirkverbundenen Zwischenglieder 17 und 18 sind bei den vorstehend erwähnten Bewegungen in entsprechend ausgebildeten Rundlöchern oder Ausnehmungen 32.1 und 32.2 des ersten Klemmarms 10 geführt. Die mit dem ersten Druckstück 26 und dem ersten Klemmarm 20 wirkverbundenen Zwischenglieder 27 und 28 sind bei den vorstehend erwähnten Bewegungen in entsprechend ausgebildeten Rundlöchern oder Ausnehmungen 36.1 und 36.2 des zweiten Klemmarms 20 geführt. Die in dem ersten Klemmarm 10 angeordneten Ausnehmungen 32.1 und 32.2 sowie die in dem zweiten Klemmarm 20 angeordneten Ausnehmungen 36.1 und 36.2 sind beispielsweise, wie in Fig.1 schematisch dargestellt, als in Längsrichtung der Klemmarme 10 und 20 orientierte Langlöcher oder Ausnehmungen ausgebildet.

Bei den vorstehend in Verbindung mit den Figuren 3 und 4 beschriebenen Bewegungen der einzelnen Elemente sind die beiden zylindrischen Zwischenglieder 17,18 in den Löchern 32.1 und 32.2 des Klemmarms 10 und die beiden zylindrischen Zwischenglieder 27,28 in den Löchern 36.1 und 36.2 des Klemmarms 20 geführt.

Die beiden plättchenförmig ausgebildeten Druckstücke 16 und 26 sind zur Erreichung einer sicheren Handhabung an der Oberfläche vorzugsweise mit einer Riffelung 19 und 29 versehen. Die Riffeiung 19,29 ist vorzugsweise quer zur Längsrichtung der Klemmarme 10 und 20 beziehungsweise quer zur Längsrichtung der Druckstücke 16,26 angeordnet (Fig.1).

Wie in Fig.5 weiterhin dargestellt, sind die beiden Klemmarme 10 und 20 an der den beiden Druckstücken 16 und 26 der Betätigungseinheit 30 zugewandten Aussenfläche jeweils mit einer Ausnehmung 31 beziehungsweise mit einer Ausnehmung 35 versehen. Die beiden aussenseitig an den Klemmarmen 10 und 20 angeordneten Ausnehmungen 31 und 35 sind vorzugsweise analog der äusseren Formgebung der zugeordneten Druckstücke 16,26 ausgebildet.

Die Ausnehmungen 31 und 35 sind weiterhin hinsichtlich der Tiefe so ausgebildet, dass das jeweils zugeordnete Druckstück 16,26 in zusammengedrücktem Zustand der beiden Klemmarme 10,20 und gespreiztem Zustand der beiden Klemmteile 15,25 (Fig.4) mindestens teilweise versenkt in der Ausnehmung 31 oder 35 angeordnet ist. Hiermit wird zudem erreicht, dass die beiden Klemmteile 15 und 25 in der Endstellung einen entsprechend grossen Gegenstand 41 ergreifen und halten können.

Bei einer nicht dargestellten Ausführungsform besteht die Möglichkeit, dass die beiden Druckstücke 16,26 als kreisrunde und mit der Riffelung 19,29 versehene Plättchen ausgebildet sind, welche je mit einem daran befestigten zylindrischen Zwischenglied 17,27 an dem jeweils zugeordneten Klemmarm 10 beziehungsweise 20 befestigt und damit wirkverbunden sind.

Die vorstehend in Verbindung mit den Figuren 1 bis 5 beschriebene und zur Erreichung einer inversen Bewegungsfunktion ausgebildete Betätigungseinheit 30 mit den einzelnen Funktionselementen ist nicht auf das dargestellte ophthalmologische Klemminstrument 5 beschränkt. Ohne den Grundgedanken der Erfindung zu verlassen sind weitere zweckmässige Ausgestaltungen und Anwendungsbeispiele desselben ebenfalls möglich.

## Patentansprüche

1. Chirurgisches Instrument, insbesondere ophthalmologisches Klemminstrument (5) mit zwei etwa parallel zueinander orientierten länglichen Klemmarmen (10,20), welche an dem hinteren Basisteil (2) miteinander wirkverbunden und an dem vorderen Funktionsteil (1) mit relativ zueinander bewegbaren Klemmteilen (15,25) versehen sind, **gekennzeichnet durch** zwei im Abstand zu dem vorderen Funktionsteil (1) gegenüberliegend zueinander an den Klemmarmen (10,20) angeordnete Druckstücke (16,26), mittels welcher beim Zusammendrücken derselben eine inverse Bewegungsfunktion und infolge davon ein Spreizen der beiden vorderen Klemmteile (15,25) entgegen der federelastischen Rückstellkraft der beiden Klemmarme (10,20) erreichbar ist.

2. Chirurgisches Instrument nach Anspruch 1, **dadurch gekennzeichnet, dass** die beiden Druckstücke (16,26) in der Ruhestellung der relativ zueinander bewegbaren Klemmteile (15,25) in weitgehend parallelem Abstand zu dem jeweils zugeordneten Klemmarm (10,20) angeordnet sind.

3. Chirurgisches Instrument nach Anspruch 1, **dadurch gekennzeichnet, dass** an jedem Druckstück (16,26) mindestens ein den zugeordneten Klemmarm (10,20) durchdringendes Zwischenglied (17,27) befestigt und jeweils mit dem gegenüberliegend angeordneten Klemmarm wirkverbunden ist.

4. Chirurgisches Instrument nach Anspruch 3, **dadurch gekennzeichnet, dass** die Zwischenglieder (17,27) jeweils durch eine Schraub- oder Nietverbindung (33.3,33.4;34.3,34.4) an den beiden Druckstücken (16,26) sowie an den beiden Klemmarmen (10,20) befestigt sind.

5. Chirurgisches Instrument nach Anspruch 1, **dadurch gekennzeichnet, dass** an jedem Druckstück (16,26) zwei den zugeordneten Klemmarm (10,20) durchdringende und in Längsrichtung desselben im Abstand zueinander angeordnete Zwischenglieder (17,18;27,28) befestigt und jeweils mit dem gegenüberliegend angeordneten Klemmarm wirkverbunden sind.

6. Chirurgisches Instrument nach Anspruch 5, **dadurch gekennzeichnet, dass** die Zwischenglieder (17,18;27,28) jeweils durch eine Schraub- oder Nietverbindung (33.1 bis 33.4;34.1 bis 34.4) an den beiden Druckstücken (16,26) sowie den beiden Klemmarmen (10,20) befestigt sind.

7. Chirurgisches Instrument nach Anspruch 3, **dadurch gekennzeichnet, dass** das eine an dem ersten Druckstück (16) angeordnete Zwischenglied (17) in einer Ausnehmung (32.2) des ersten Klemmarms (10) und das andere an dem gegenüberliegenden zweiten Druckstück (26) angeordnete Zwischenglied (27) in einer Ausnehmung (36.2) des zweiten Klemmarms (20) geführt ist.

8. Chirurgisches Instrument nach Anspruch 5, **dadurch gekennzeichnet, dass** die beiden im Abstand zueinander an dem ersten Druckstück (16) angeordneten Zwischenglieder (17,18) je in einer Ausnehmung (32.1,32.2) des ersten Klemmarms (10) und die beiden anderen im Abstand zueinander an dem zweiten Druckstück (26) angeordneten Zwischenglieder (27,28) je in einer Ausnehmung (36.1,36.2) des zweiten Klemmarms (20) geführt sind.

9. Chirurgisches Instrument nach Anspruch 1, **dadurch gekennzeichnet, dass** der erste Klemmarm (10) auf der dem ersten Druckstück (16) zugewandten Seite mit einer ersten Ausnehmung (31) und der zweite Klemmarm (20) auf der dem zweiten Druckstück (26) zugewandten Seite mit einer zweiten Ausnehmung (35) versehen ist.

10. Chirurgisches Instrument nach Anspruch 9, **dadurch gekennzeichnet, dass** die aussenseitig an den beiden Klemmarmen (10,20) vorgesehene Ausnehmung (31,35) jeweils analog der äusseren Formgebung des zugeordneten und aussenseitig mit einer Riffelung (19,29) versehenen Druckstücks (16,26) und zur Aufnahme desselben ausgebildet ist.
